# FASCICULE DE BREVET EUROPEEN

(11) **EP 2 018 417 B1**
(45) Date de publication et mention de la délivrance du brevet: **02.03.2011**
(21) Numéro de dépôt: 07731332.8
(22) Date de dépôt: 20.04.2007
(51) Int. Cl.: C12N 1/20, C02F 3/34, C02F 3/12

(54) **NOUVEAU MICRO-ORGANISME POUR LE TRAITEMENT DES EAUX USÉES ET PROCÉDÉ**
NEUER MIKROORGANISMUS ZUR BEHANDLUNG VON ABWASSER UND DAZUGEHÖRIGES VERFAHREN
NOVEL MICROORGANISM FOR TREATING WASTEWATER AND CORRESPONDING METHOD

(30) Priorité: 27.04.2006 FR 0603792
(43) Date de publication de la demande: 28.01.2009
(73) Titulaire: Eco Solution, 93230 Romainville (FR)
(72) Inventeur: DA COSTA, Alexandre, 77176 Savigny le Temple (FR)
(74) Mandataire: Galup, Cédric Olivier Nicolas
(86) Numéro de dépôt international: PCT/FR2007/000669
(87) Numéro de publication internationale: WO 2007/128897

(56) Documents cités:
- GB-A- 2 030 557
- JOO HUNG-SOO ET AL: "Nitrification and denitrification in high-strength ammonium by Alcaligenes faecalis" BIOTECHNOLOGY LETTERS, vol. 27, no. 11, juin 2005 (2005-06), pages 773-778, XP009074254 ISSN: 0141-5492

## Description

### Domaine technique

La présente invention entre dans le cadre de la dépollution des eaux usées. Elle concerne en particulier l'isolement et la caractérisation d'un nouveau microorganisme capable de transformer la matière carbonée et la matière azotée en aérobiose. Ce microorganisme est particulièrement utile pour le traitement des effluents chargés en carbone et en azote par voie biologique.

### Art antérieur

Actuellement, le traitement de la matière carbonée et azotée par voie biologique passe par l'utilisation d'au moins deux types de microorganismes, les premiers spécialisés dans le traitement de la matière carbonée, les deuxièmes spécialisés dans le traitement de la matière azotée.

En effet, la dépollution de la matière organique se produit généralement dans des bassins à boues activées dans lesquels la matière carbonée est transformée, en aérobiose, en biomasse et dioxyde de carbone. Alternativement, la matière organique peut être transformée en biomasse et en méthane en anaérobiose.

Après l'un ou l'autre de ces traitements, la charge azotée des effluents en cours de traitement reste importante. Le traitement de la charge azotée nécessite une étape ultérieure impliquant des microorganismes spécialisés.

Il existe différents procédés exploités à ce jour pour transformer l'azote sous forme ammoniacale (NH₃) ou oxydée (NO₂⁻, NO₃⁻) contenu dans les effluents aqueux en azote gazeux (N₂).

Le traitement le plus répandu à l'heure actuelle est constitué d'une étape de nitrification aérobie suivi d'une étape de dénitrification anoxique.

La nitrification consiste en l'oxydation de l'azote ammoniacal (NH₃) en nitrites (NO₂⁻) grâce à des bactéries autotrophes incluant, par exemple, des espèces des genres Nitrosomonas, Nitrosospira et Nitrosococcus, puis en l'oxydation des nitrites produits précédemment en nitrates (NO₃⁻) grâce à des bactéries autotrophes incluant par exemple des espèces des genres Nitrobacter, Nitrococcus et Nitrospira.

La dénitrification consiste en la réduction des formes oxydées de l'azote minéral (nitrites (NO₂⁻), nitrates (NO₂⁻)) en composés gazeux (N₂), par une population microbienne fonctionnelle dénitrifiante, la plupart du temps hétérotrophe, en présence d'un donneur d'électrons. Ce processus est réalisé par de nombreuses bactéries incluant, par exemple, des espèces appartenant aux genres Pseudomonas, Bacillus, Paracoccus, Thiobacillus, Alcaligenes. La réduction des formes oxydées de l'azote est donc couplée avec l'oxydation d'un composé organique. Cela signifie que le carbone est un facteur limitant la dénitrification. En effet, si la concentration en matière carbonée des effluents aqueux à traiter n'est pas suffisante pour maintenir les populations microbiennes actives vis-à-vis du traitement de l'azote, l'ajout d'une source de carbone exogène peut se révéler nécessaire au bon fonctionnement des réacteurs de dénitrification. C'est le cas en particulier, si l'effluent à traiter est peu chargé en carbone (rapport Carbone/Azote (C/N) < 4), ou s'il subit une décantation primaire avant dénitrification.

La dénitrification peut être réalisée en traitant l'effluent collecté après l'étape de nitrification dans un bassin d'anoxie contenant les bactéries dénitrifiantes.

Selon un autre mode de fonctionnement, le bassin de nitrification peut être périodiquement exploité comme un bassin de dénitrification en le rendant anoxique, par exemple en faisant des interruptions d'apport d'oxygène et en ajoutant des donneurs d'électrons parmi lesquels le méthanol est le plus souvent utilisé.

Parmi les inconvénients de cette méthode de nitrification-dénitrification, on notera que les souches nitrifiantes autotrophes possèdent un faible taux de croissance. Cette caractéristique cinétique induit des temps de séjours importants dans les bassins ce qui conduit à la nécessité soit de concevoir de grands ouvrages, soit de rallonger la chaîne de traitement par de nouveaux ouvrages ce qui entraîne des coûts d'investissement élevés. De plus, dans le cas d'une étape de dénitrification conduite dans un bassin additionnel, il est nécessaire d'apporter une source de carbone organique à la flore dénitrifiante, telle que le méthanol, ce qui peut engendrer des surcoûts d'exploitation.

Un autre procédé de traitement de l'azote ammoniacal appelé Anammox (pour Anaerobic Ammonia Oxydation) permet la bioconversion de l'azote ammoniacal et des nitrites en azote gazeux. Cette réaction peut être réalisée en traitant l'effluent de nitrification (contenant des nitrites et de l'ammonium n'ayant pas réagi) dans un bassin séparé pour produire l'azote gazeux. Selon un autre mode de fonctionnement, le bassin de nitrification peut être conduit en alternant des conditions oxiques et anoxiques, ou en continu selon des conditions de limitation en oxygène. Dans ce cas, la consommation d'oxygène par les bactéries nitrifiantes génère les conditions anoxiques du procédé Anammox. Les bactéries capables de catalyser la réaction Anammox peuvent être obtenues à partir de boues activées classiques, dans la mesure où celles-ci contiennent des bactéries planctomycètes du type *Brocardia anammoxidans.*

Le principal inconvénient du procédé Anammox est le faible taux de croissance des bactéries planctomycètes utilisées, ce qui entraîne des phases de démarrage d'installations importantes. De surcroît, les conditions de culture de ces bactéries et leur maintien en cultures pures demeurent difficiles. Enfin, la nitrification partielle (conversion de 50% de l'ammoniac en nitrites) doit être parfaitement maîtrisée, ce qui est difficile à réaliser en condition d'exploitation réelle.

Le procédé Sharon placé en amont du traitement Anammox permet l'obtention d'une nitrification partielle. Le procédé Sharon est basé sur la différence qu'il existe entre le taux de croissance des bactéries oxydant l'ammoniac et celui des bactéries oxydant les nitrites. Ce traitement fonctionne à un temps de rétention hydraulique inférieur au taux de croissance des bactéries oxydant les nitrites, mais plus élevé que celui des bactéries oxydant l'ammoniac (près d'un jour). Puisque il n'y a pas de rétention de boues, les bactéries oxydant les nitrites ne se maintiennent pas dans le réacteur et sont donc éliminées.

Récemment, des souches d'*Alcaligenes faecalis* ont été identifiées comme capables de réaliser la nitrification et la dénitrification en aérobiose. C'est le cas de la souche *Alcaligenes faecalis* sp. No. 4 décrite dans l'article de Joo et al. (Biotechnology Letters (2005) 27:773-778). Grâce à une telle souche, le traitement de l'azote ammoniacal peut être réalisé en une seule étape aérobie.

Cependant, cette bactérie ne permet pas de transformer la totalité de l'azote ammoniacal en azote gazeux. En effet, des quantités non négligeables de produits intermédiaires de dénitrification s'accumulent. Ainsi, dans le meilleur des cas, le traitement de fortes teneurs en ammonium basé sur l'utilisation d*'Alcaligenes faecalis* sp. No. 4 en aérobiose, avec un rapport C/N de 10, conduit à éliminer 40-50% de NH₄⁺ par dénitrification et 90% des produits de dénitrification sont de l'azote gazeux. D'autre part, cette bactérie fonctionne de manière optimale avec des rapports C/N élevés, nécessitant une supplémentation de l'effluent à traiter en carbone (Joo *et al*. (2005)).

Par conséquent, l'amélioration des performances du traitement de l'azote ammoniacal dans les eaux usées passe aujourd'hui par l'ajout aux installations existantes de traitements spécifiques dits traitements tertiaires, à boues activées ou à biofiltres. Les équipements et ouvrages nécessaires pour conduire ces traitements représentent des investissements conséquents et génèrent également des coûts d'exploitation significatifs lorsqu'il devient nécessaire d'ajouter des substrats carbonés par exemple lors dé la dénitrification.

La présente invention vise à pallier les inconvénients de l'art antérieur et décrit un mode de traitement de la matière carbonée et de l'azote sous forme Kjeldahl, ammoniacale ou oxydée par voie biologique au sein d'une unique étape aérobie par des micro-organismes hétérotrophes possédant une vitesse de croissance importante qui permet de concevoir des ouvrages d'épuration de taille réduite.

L'invention vise également à obtenir une réduction des coûts d'exploitation des stations d'épuration notamment celles qui utilisent des bassins de dénitrification placés en aval des bassins de nitrification. En effet, puisque l'azote ammoniacal est transformé en azote gazeux dans les bassins d'aération, les bassins de dénitrification anoxiques placés en aval n'ont plus d'utilité.

De ce fait, l'économie générée provient d'une part de la suppression de l'ajout de substrat carboné organique (type méthanol), et d'autre part, de la réduction de production de boues par élimination de l'étape de traitement anoxique.

L'invention permet donc une exploitation optimale des installations existantes de traitement des eaux sans nécessiter d'étapes de traitements complémentaires.

### Description de l'invention

Dans ce contexte, le demandeur a isolé un nouveau microorganisme capable de transformer la matière carbonée et la matière azotée en aérobiose qui le rend particulièrement utile dans le traitement des eaux usées.

La matière azotée transformée par le microorganisme selon l'invention peut être l'azote Kjeldahl, l'azote ammoniacal et/ou des oxydes d'azote.

L'azote Kjeldahl comprend l'azote sous forme organique et sous forme ammoniacale à l'exclusion des formes nitreuses (nitrites, nitrates).

Ainsi l'invention concerne un micro-organisme isolé caractérisé en ce qu'il est capable de réaliser :
i) la transformation de l'azote Kjeldahl, de l'azote ammoniacal et/ou des oxydes d'azote en azote gazeux ; et
ii) la transformation de la matière carbonée en dioxyde de carbone ;
les deux transformations se déroulant en aérobiose.

L'invention a donc pour objet premier un microorganisme isolé caractérisé en ce qu'il s'agit de la souche CNCM I-3448 d'*Alcaligenes faecalis* capable de réaliser :
i) la transformation de l'azote Kjeldahl, de l'azote ammoniacal et/ou des oxydes d'azote en azote gazeux ; et
ii) la transformation de la matière carbonée en dioxyde de carbone ;
les deux transformations se déroulant en aérobiose.

De manière préférée, le micro-organisme selon l'invention est capable de transformer l'azote Kjeldahl, l'azote ammoniacal et/ou des oxydes d'azote en azote gazeux en accumulant moins de 1% d'oxydes d'azote.

De manière encore préférée, le micro-organisme selon l'invention est capable d'effectuer la transformation de l'azote Kjeldahl, de l'azote ammoniacal et/ou des oxydes d'azote en azote gazeux en accumulant moins de 1% d'oxydes d'azote, ladite transformation étant optimale lorsque le rapport C/N du milieu est inférieur à 4, de préférence inférieur à 3, de préférence de l'ordre de 1,5.

Le microorganisme selon l'invention est capable d'effectuer la transformation de l'azote Kjeldahl, de l'azote ammoniacal et/ou des oxydes d'azote en azote gazeux dans des conditions de charge massique appliquée (CMA) inférieure à 0,2 kg _{DBO5}/kg _{MVS}/j.

Est plus particulièrement préféré un micro-organisme isolé tel que décrit précédemment, caractérisé en ce qu'il transforme l'azote Kjeldahl, l'azote ammoniacal et/ou des oxydes d'azote dans des conditions de charge massique appliquée (CMA) supérieure ou égale à 0,2 kg _{DBO5}/kg _{MVS}/j, de préférence supérieure à 0,5 kg _{DBO5}/kg _{MVS}/j.

Le microorganisme selon l'invention appartient au genre Alcaligenes, en particulier à l'espèce *Alcaligenes faecalis.*

Le micro-organisme selon l'invention est représenté par la souche *Alcaligenes faecalis* déposée le 10 juin 2005 à la Collection Nationale de Cultures de Microorganismes auprès de l'Institut Pasteur (CNCM) sous la référence CNCM I-3448.

L'invention décrit des micro-organismes dérivés de la souche CNCM I-3448, ayant la capacité de transformer la matière carbonée et la matière azotée en aérobiose.

Par micro-organisme dérivé, on entend tout micro-organisme issu de la souche CNCM I-3448 qui pourrait résulter, par exemple, d'une étape de culture, de mutation, de transformation de la souche CNCM I-3448 ou même de son croisement avec un autre micro-organisme, et qui aurait conservé les caractéristiques essentielles de la souche CNCM I-3448.

Un autre objet de l'invention est l'utilisation du micro-organisme selon l'invention pour le traitement des eaux usées.

L'utilisation préférée du micro-organisme selon l'invention concerne la transformation de l'azote Kjeldahl, de l'azote ammoniacal et/ou des oxydes d'azote contenus dans les eaux usées en azote gazeux, en aérobiose.

Selon un aspect de l'utilisation selon l'invention, moins de 1% d'oxydes d'azote (NO₃⁻, NO₂⁻) s'accumulent.

De manière encore plus préférée, l'utilisation du micro-organisme selon l'invention concerne les traitements combinés de l'azote Kjeldahl, de l'azote ammoniacal et/ou des oxydes d'azote et de la matière carbonée contenus dans les eaux usées, en aérobiose.

En particulier, lors de l'utilisation selon l'invention, l'azote Kjeldahl, l'azote ammoniacal et/ou des oxydes d'azote sont transformés en azote gazeux et la matière carbonée est transformée en dioxyde de carbone.

L'utilisation selon l'invention peut s'effectuer dans des conditions où la charge massique appliquée (CMA) du milieu est inférieure à 0,2 kg _{DBO5}/kg _{MVS/}j.

De préférence, l'utilisation selon l'invention s'effectue dans des conditions où la charge massique appliquée (CMA) est supérieure à 0,2 kg _{DBO5}/kg _{MVS}/j, de manière encore plus préférée supérieure à 0,5 kg _{DBO5}/kg _{MVS/}j.

Selon un mode de réalisation préféré de l'utilisation selon l'invention, aucune source carbonée externe n'est ajoutée aux eaux usées à traiter.

Une des caractéristiques préférée de l'utilisation selon l'invention consiste en ce que les traitements combinés de l'azote Kjeldahl, de l'azote ammoniacal et/ou des oxydes d'azote et de la matière carbonée contenus dans les eaux usées se déroulent dans un unique bassin de boues activées libres.

Alternativement, l'utilisation selon l'invention est caractérisée par le fait que les traitements combinés de l'azote Kjeldahl, de l'azote ammoniacal et/ou des oxydes d'azote et de la matière carbonée contenus dans les eaux usées se déroulent dans un dispositif à cultures fixées sur un support.

Selon un autre aspect, l'invention concerne l'utilisation d'un microorganisme selon l'invention, préalablement produit par culture continue. De préférence, ladite culture continue est réalisée dans des conditions stériles. De manière encore plus préférée, ladite culture continue est réalisée sous pression de sélection et sur des durées indéfinies.

Un autre objet de l'invention concerne un procédé de traitement des eaux usées caractérisé en ce qu'il met en oeuvre un microorganisme selon l'invention.

Selon un aspect de l'invention, le procédé de traitement des eaux usées comprend les étapes suivantes :
i) la culture du microorganisme selon l'invention ;
ii) l'alimentation automatique d'un dispositif de traitement contenant les eaux usées à traiter par des apports répétitifs de la culture produite à l'étape i) ; et
iii) le traitement biologique des matières carbonée et azotée contenues dans les eaux usées, au sein du dispositif de traitement, en aérobiose.

Selon un aspect de l'invention, le traitement biologique de la matière carbonée et le traitement biologique de la matière azotée de l'étape iii) se déroulent simultanément.

De manière préférée, le dispositif de traitement des eaux usées mentionné aux étapes ii) et iii) est un bassin d'aération.

L'invention a également pour objet le contrôle de l'efficacité de la dépollution biologique des eaux usées de manière à optimiser le traitement des effluents en fonction des contraintes, par exemple, l'augmentation de charge carbonée et ou azotée.

Aussi, de manière encore plus préférée, l'activité du microorganisme selon l'invention présent au sein des boues activées libres du bassin d'aération selon l'un des procédés décrit ci-dessus est évaluée par des transferts réguliers de prélèvements vers un dispositif de culture puis comparée avec une culture dudit microorganisme maintenue sous pression de sélection.

Selon un autre mode de réalisation, le procédé selon l'invention comprend, en parallèle des transformations aérobies de l'azote Kjeldahl, de l'azote ammoniacal et/ou des oxydes d'azote et de la matière carbonée par le microorganisme selon l'invention dans le dispositif de traitement, la culture en continu du microorganisme selon l'invention dans un milieu contenant des nutriments (source de carbone, source d'azote...) nécessaires à la croissance microbienne afin d'inoculer périodiquement le dispositif de traitement avec ledit microorganisme.

Préférentiellement, la croissance microbienne est réalisée dans un dispositif permettant de cultiver le microorganisme selon l'invention dans des conditions de culture stérile. Plus particulièrement, cette culture est réalisée sous pression de sélection et sur des durées indéfinies.

Selon un mode particulier de réalisation de l'invention, cette culture parallèle est utilisée pour ensemencer régulièrement un réacteur d'amplification ayant un volume de quelques centaines de litres à quelques mètres cubes, dans lequel une culture du microorganisme selon l'invention est produite non stérilement en quantité importante. Le milieu de culture peut être synthétique ou consister en un effluent à traiter et doit être suffisamment sélectif pour favoriser la production importante dudit microorganisme. Des apports répétitifs de cette culture pourront être automatiquement réalisés selon une fréquence définie vers le dispositif de traitement des eaux usées, par exemple le bassin d'aération à boues activées libres. Aucun substrat organique comme par exemple le méthanol n'est ajouté dans le dispositif de traitement pour favoriser le maintien du microorganisme selon l'invention étant donné que celui-ci utilise comme source de carbone la matière organique présente dans les eaux usées à traiter.

Alternativement, de manière avantageuse, le dispositif de traitement des eaux usées mentionné aux étapes ii) et iii) est un dispositif à cultures fixées sur un support.

Selon un autre aspect du procédé selon l'invention, ledit procédé comporte, en amont du dispositif de traitement, une étape d'évaluation de la toxicité des eaux usées entrant vis-à-vis du microorganisme. L'étape d'évaluation de la toxicité peut être basée, en particulier, sur la mesure de la croissance dudit microorganisme sur lesdites eaux usées.

Un effluent toxique pour le microorganisme, et donc néfaste à la transformation de l'azote Kjeldahl, de l'azote ammoniacal et/ou des oxydes d'azote en azote gazeux, pourrait alors être orienté vers un bassin de sécurité pour un traitement ultérieur.

Selon un autre aspect de l'invention, le microorganisme est fixé sur un support.

Ledit support peut contenir un garnissage de type structuré ou autre, permettant d'optimiser l'implantation de la population cultivée du microorganisme selon l'invention, sa fixation et son développement dans des conditions favorables. Ce garnissage peut être de différents types et de différents matériaux connus de l'homme de l'art.

En particulier, le microorganisme selon l'invention est fixé seul ou en co-culture avec un ou plusieurs microorganismes spécialisés dans le traitement de la pollution carbonée.

La répartition du micro-organisme selon l'invention à la surface du support doit être la meilleure possible et le bon déroulement du procédé dépend des transferts entre les micro-organismes et l'effluent à traiter. En termes d'aération, l'installation doit être conçue pour que le transfert d'oxygène soit le plus efficace possible pour les micro-organismes épurateurs fixés sur le support. D'autre part, il faut un bon transfert de l'effluent vers les micro-organismes fixés pour ce qui est des polluants à traiter (notamment l'azote Kjeldahl, l'azote ammoniacal et/ou les oxydes d'azote et les matières carbonées), et en sens inverse pour les produits du métabolisme des micro-organismes (notamment le dioxyde de carbone et l'azote gazeux).

Selon l'invention, les eaux usées à traiter incluent toute eau usée qu'elle soit municipale, industrielle, agricole ou de toute autre origine, qui contient des valeurs significatives d'azote ammoniacal. La teneur en azote Kjeldahl ou ammoniacal de l'effluent à traiter est de, par exemple, 50 mg/l, notamment 1 g/l, particulièrement 30 g/l. Les eaux usées à traiter peuvent également provenir de l'eau utilisée pour épurer des gaz contenant de l'azote ammoniacal.

Le procédé selon l'invention est particulièrement adapté au traitement des eaux usées contenant plus de 30 g/l d'azote Kjeldahl ou d'azote ammoniacal.

Les eaux usées à traiter contiennent également des valeurs significatives de carbone organique. Les teneurs en carbone organique total peuvent être, par exemple, de 80 mg/l, notamment 1 g/l, particulièrement 80 g/l ou 95 g/l.

Selon un autre aspect, le procédé selon l'invention s'accompagne de l'accumulation de moins de 1% d'oxydes d'azote (NO₂⁻, NO₃⁻).

Les exemples qui suivent illustrent l'invention mais ceux-ci ne sauraient être considérés comme limitatifs.

### Exemple 1 : Bioconversion d'un effluent synthétique urbain

Un fermenteur de 1 litre utile est alimenté en continu avec un effluent synthétique reprenant la composition moyenne d'eaux usées d'origine urbaine. La composition de cet effluent est la suivante : 53 mg/l de N-NH₃ sous la forme de (NH₄)₂SO₄, 82 mg/l de carbone organique total (COT) sous la forme d'acétate de sodium, 0,1 g/l de K₂HPO₄ et des micro-éléments connus de l'homme de l'art pour favoriser la croissance de ces micro-organismes. Le pH de la solution est ajusté à 8 avec HCl/NaOH si nécessaire.

Le fermenteur contient une culture pure de la souche *Alcaligenes faecalis* CNCM I-3448.

Le régime continu est maintenu en fixant une charge volumique appliquée de 1,9 g NH₃/m³/j et 2,6 g C/m³/j.

Ce mode de réalisation correspond à des conditions où la charge massique appliquée (CMA) est de 0,27 kg _{DBO5}/kg _{MVS}/j et le rapport C/N du milieu est de 1,5.

L'aération de la culture est assurée par barbotage d'air comprimé (concentration en oxygène dissout supérieure à 3 mg/l). L'unique sortie d'air est reliée à un barboteur contenant 2 litres d'eau osmosée à pH 5. L'objet de ce montage est de s'assurer que de l'ammoniac n'est pas évacué par l'aération lors de la biodégradation en continu de l'effluent.

Lorsque le régime stabilisé est atteint, la concentration en ammoniac dans le barboteur (pH 5) est nulle, la concentration moyenne en matière sèche est de 0,7 g/l, 90% du carbone (COT) entrant est soit converti dans la biomasse soit en dioxyde de carbone.

On mesure un abattement de 99,9% sur l'azote ammoniacal qui est converti soit dans la biomasse soit en azote gazeux. Le rendement de bioconversion sur l'azote obtenu est donc supérieur à 99,9 %.

Par ailleurs, on ne mesure pas d'accumulation de nitrites et de nitrates dans le milieu, ni dans l'effluent en sortie.

### Exemple 2 : Bioconversion d'effluents industriels concentrés en azote organique

On alimente en continu un fermenteur de 7,5 litres utiles avec des eaux usées comprenant essentiellement des solvants azotés (comme l'acétonitrile ou la diméthylformamide) issus de la production de peptides.

L'analyse de l'effluent révèle une concentration en carbone organique total (COT) de 95 g/l et une concentration en azote Kjeldahl de 34 g/l (provenant essentiellement de l'acétonitrile).

Le fermenteur contient une culture pure de la souche *Alcaligenes faecalis* CNCM I-3448.

Le régime continu est maintenu en fixant une charge volumique appliquée de 1,2 kg N/m³/j et 3,2 kg C/m³/j.

Ce mode de réalisation correspond à des conditions où la charge massique appliquée (CMA) est de 0,96 kg _{DBO5}/kg _{MVS}/j et le rapport C/N du milieu est de 2,8.

L'air entrant dans la culture (8 l/min) passe par un humidificateur. L'unique sortie d'air est reliée à un barboteur contenant 2 litres d'eau osmosée à pH 5. L'objet de ce montage est de s'assurer que de l'azote Kjeldahl n'est pas évacué par l'aération lors de la biodégradation en continu de l'effluent. On observe que le flux d'azote Kjeldahl dans les effluents gazeux représente le dixième du flux entrant.

Lorsque le régime stabilisé est atteint, la concentration en azote Kjeldahl dans le barboteur (pH 5) est nulle, la concentration moyenne en matière sèche est de 10 g/l, 98% du carbone (COT) entrant est soit converti dans la biomasse soit en dioxyde de carbone.

On mesure un abattement de 99,9% sur l'azote Kjeldahl qui est converti soit dans la biomasse soit en azote gazeux.

Par ailleurs, l'accumulation dans le milieu et dans l'effluent de sortie des formes oxydées de l'azote (NO₂⁻ et NO₃⁻) est inférieure à 1% et temporaire (< 12 h).

Le rendement de bioconversion sur l'azote Kjeldahl obtenu est donc supérieur à 99 %.

## Revendications

1. Microorganisme isolé **caractérisé en ce qu'**il s'agit de la souche CNCM I-3448 d*'Alcaligenes faecalis* capable de réaliser:
i) la transformation de l'azote Kjeldahl, de l'azote ammoniacal et/ou des oxydes d'azote en azote gazeux ; et
ii) la transformation de la matière carbonée en dioxyde de carbone;
les deux transformations se déroulant en aérobiose.

2. Microorganisme isolé selon la revendication 1, **caractérisé en ce qu'**il transforme l'azote Kjeldahl, l'azote ammoniacal et/ou des oxydes d'azote dans des conditions de charge massique appliquée (CMA) supérieure ou égale à 0,2 kg _{DBO5}/kg _{MVS}/j, préférentiellement supérieure à 0,5 Kg _{DBO5}/kg _{MVS}/j..

3. Microorganisme selon l'une quelconque des revendications 1 ou 2, **caractérisé en ce qu'**il transforme l'azote Kjeldahl, l'azote ammoniacal et/ou des oxydes d'azote en azote gazeux en accumulant moins de 1% d'oxydes d'azote.

4. Microorganisme selon l'une quelconque des revendications 1 à 3, **caractérisé en ce qu'**il transforme l'azote Kjeldahl, l'azote ammoniacal et/ou des oxydes d'azote en azote gazeux de manière optimale lorsque le rapport C/N dans le milieu est inférieur à 4, de préférence inférieure à 3, de manière encore plus préférée de l'ordre de 1,5.

5. Utilisation du microorganisme selon l'une quelconque des revendications 1 à 4, pour le traitement des eaux usées.

6. Utilisation selon la revendication 5, **caractérisée en ce que** la charge massique appliquée (CMA) est supérieure à 0,2 kg _{DBO5}/kg _{MVS}/j, préférentiellement supérieure à 0,5 kg _{DBO5}/kg _{MVS}/j.

7. Utilisation selon l'une quelconque des revendications 5 ou 6, **caractérisée en ce qu'**aucune source carbonée externe n'est ajoutée aux eaux usées à traiter.

8. Procédé de traitement des eaux usées **caractérisé en ce qu'**il met en oeuvre un microorganisme selon l'une quelconque des revendications 1 à 4.

9. Procédé de traitement des eaux usées selon la revendication 8 comprenant les étapes suivantes :
i) la culture du microorganisme selon l'une des revendications 1 à 5 ;
ii) l'alimentation automatique d'un dispositif de traitement contenant les eaux usées à traiter par des apports répétitifs de la culture produite à l'étape i) ; et
iii) le traitement biologique des matières carbonée et azotée contenues dans les eaux usées, au sein du dispositif de traitement, en aérobiose.

10. Procédé de traitement des eaux usées selon la revendication 9, dans lequel le traitement biologique de la matière carbonée et le traitement biologique de la matière azotée de l'étape iii) se déroulent simultanément.

11. Procédé selon l'une quelconque des revendications 9 ou 10, dans lequel le dispositif de traitement des eaux usées est un bassin d'aération à boues activées libres.

12. Procédé selon l'une quelconque des revendications 9 ou 10, dans lequel le dispositif de traitement des eaux usées est un dispositif à cultures fixées sur un support.

13. Procédé selon l'une des revendications 8 à 12, dans lequel les eaux usées à traiter contiennent plus de 30 g/l d'azote Kjeldahl ou d'azote ammoniacal.

## Claims

1. Isolated microorganism, **characterized in that** it is the strain CNCM I-3448 of *Alcaligenes faecalis*, capable of carrying out:
i). the conversion of Kjeldahl nitrogen, ammonia nitrogen and/or nitrogen oxides into gaseous nitrogen; and
ii). conversion of the carbon-containing matter into carbon dioxide;
both conversions taking place under aerobic conditions.

2. Isolated microorganism according to claim 1, **characterized in that** it converts Kjeldahl nitrogen, ammonia nitrogen and/or nitrogen oxides under conditions of applied mass loading (AML) greater than or equal to 0.2 kg _{BOD5}/kg_{VMS}/d,preferably greater than 0.5 kg _{BOD5}/kg_{VMS}/d.

3. Microorganism according to either one of claims 1 or 2, **characterized in that** it converts Kjeldahl nitrogen, ammonia nitrogen and/or nitrogen oxides into gaseous nitrogen whilst accumulating less than 1% nitrogen oxides.

4. Microorganism according to any one of claims 1 to 3, **characterized in that** it converts Kjeldahl nitrogen, ammonia nitrogen and/or nitrogen oxides into gaseous nitrogen optimally when the C/N ratio in the environment is less than 4, preferably less than 3, even more preferably of the order of 1.5.

5. Use of microorganisms according to any one of claims 1 to 4, for the treatment of wastewater.

6. Use according to claim 5, **characterized in that** the applied mass loading (AML) is greater than 0.2 kg _{BOD5/}kg _{VMS}/d, preferably greater than 0.5 kg _{BOD5/}kg _{VMS}/d.

7. Use according to either one of claims 5 or 6, **characterized in that** no external carbon-containing source is added to the wastewater to be treated.

8. Method for the treatment of wastewater, **characterized in that** it uses a microorganism according to any one of claims 1 to 4.

9. Method for the treatment of wastewater according to claim 8, comprising the following steps:
i). culturing the microorganism according to one of claims 1 to 5;
ii). automatically feeding a treatment device containing the wastewater to be treated by repeatedly adding the culture produced in step i);
and
iii). biologically treating carbon-containing and nitrogen-containing matter contained in the wastewater, within the treatment device, under aerobic conditions.

10. Method for the treatment of wastewater according to claim 9, in which the biological treatment of the carbon containing matter and the biological treatment of the nitrogen-containing matter in step iii) take place simultaneously.

11. Method according to either one of claims 9 or 10, in which the device for the treatment of wastewater is a free activated sludge aeration tank.

12. Method according to either one of claims 9 or 10, in which the device for the treatment of wastewater is a device with cultures fixed on a support.

13. Method according to any one of claims 8 to 12, in which the wastewater to be treated contains more than 30 g/I of Kjeldahl nitrogen or ammonia nitrogen.

## Patentansprüche

1. Isolierter Mikroorganismus, **dadurch gekennzeichnet, dass** es sich um den Stamm CNCM 1-3448 von *Alcaligenes faecalis* mit den folgenden Fähigkeiten handelt:
i). Umwandeln von Kjeldahl-Stickstoff, Ammoniumstickstoff und/oder Stickstoffoxiden in Stickstoffgas; und
ii). Umwandeln der kohlenstoffhaltigen Substanz in Kohlendioxid;
wobei die beiden Umwandlungen aerobisch ablaufen.

2. Isolierter Mikroorganismus nach Anspruch 1, **dadurch gekennzeichnet, dass** er den Kjeldahl-Stickstoff, den Ammoniumstickstoff und/oder die Stickstoffoxide unter Bedingungen einer applizierten Massenbeladung von gleich oder höher als 0,2 kg _{DBO5/}kg _{MVS}/Tag, vorzugsweise höher als 0,5 kg _{DBO5}/kg _{MVS}/Tag umwandelt.

3. Mikroorganismus nach Anspruch 1 oder 2, **dadurch gekennzeichnet, dass** er den Kjeldahl-Stickstoff, den Ammoniumstickstoff und/oder die Stickstoffoxide unter Ansammlung von weniger als 1 % Stickstoffoxiden in Stickstoffgas umwandelt.

4. Mikroorganismus nach irgendeinem der Ansprüche 1 bis 3, **dadurch gekennzeichnet, dass** er den Kjeldahl-Stickstoff, den Ammoniumstickstoff und/oder die Stickstoffoxide auf optimale Weise in Stickstoffgas umwandelt, wenn das C/N-Verhältnis in dem Medium kleiner als 1:4, vorzugsweise kleiner als 1:3, noch stärker bevorzugt etwa 1:1,5 ist.

5. Verwendung des Mikroorganismus nach irgendeinem der Ansprüche 1 bis 4 für die Behandlung von Abwasser.

6. Verwendung nach Anspruch 5, **dadurch gekennzeichnet, dass** die applizierte Massenbeladung höher als 0,2 kg _{DBO5/}kg _{MVS}/Tag, vorzugsweise höher als 0,5 kg _{DBO5}/kg _{MVS}/Tag ist.

7. Verwendung nach Anspruch 5 oder 6, **dadurch gekennzeichnet, dass** dem zu behandelnden Abwasser keine externe Kohlenstoffquelle zugegeben wird.

8. Verfahren zur Behandlung von Abwasser, **dadurch gekennzeichnet, dass** es einen Mikroorganismus nach irgendeinem der Ansprüche 1 bis 4 aktiviert.

9. Verfahren zur Behandlung von Abwasser nach Anspruch 8, das die folgenden Schritte beinhaltet:
i). Kultivieren des Mikroorganismus nach einem der Ansprüche 1 bis 5;
ii). automatisches Speisen einer Behandlungsvorrichtung, die das zu behandelnde Abwasser enthält, durch wiederholtes Einbringen der in Schritt i) erzeugten Kultur; und
iii). biologisches Behandeln der in dem Abwasser enthaltenen kohlenstoff- und stickstoffhaltigen Substanzen im Innern der Behandlungsvorrichtung auf aerobische Weise.

10. Verfahren zur Behandlung von Abwasser nach Anspruch 9, bei dem die biologische Behandlung der kohlenstoffhaltigen Substanz und die biologische Behandlung der stickstoffhaltigen Substanz aus Schritt iii) gleichzeitig ablaufen.

11. Verfahren nach Anspruch 9 oder 10, bei dem die Vorrichtung zur Behandlung von Abwasser ein Belüftungsbecken mit freien Belebtschlämmen ist.

12. Verfahren nach Anspruch 9 oder 10, bei dem die Vorrichtung zur Behandlung von Abwasser eine Vorrichtung mit auf einem Substrat fixierten Kulturen ist.

13. Verfahren nach einem der Ansprüche 8 bis 12, bei dem das zu behandelnde Abwasser mehr als 30 g/l Kjeldahl-Stickstoff oder Ammoniumstickstoff enthält.
